(19) Europäisches Patentamt
European Patent Office
Office européen des brevets

(11) Numéro de publication : **0 480 980 B1**

(12) # FASCICULE DE BREVET EUROPEEN

(45) Date de publication du fascicule du brevet :
**27.10.93 Bulletin 93/43**

(51) Int. Cl.$^5$ : **G01N 29/02**

(21) Numéro de dépôt : **90910770.8**

(22) Date de dépôt : **26.06.90**

(86) Numéro de dépôt international :
**PCT/FR90/00472**

(87) Numéro de publication internationale :
**WO 91/00516 10.01.91 Gazette 91/02**

(54) **PROCEDE ET DISPOSITIF DE MESURE DE LA GELIFICATION DE PRODUITS PETROLIERS PARAFFINIQUES, NOTAMMENT BRUTS.**

(30) Priorité : **03.07.89 FR 8908888**

(43) Date de publication de la demande :
**22.04.92 Bulletin 92/17**

(45) Mention de la délivrance du brevet :
**27.10.93 Bulletin 93/43**

(84) Etats contractants désignés :
**BE DE ES GB IT NL**

(56) Documents cités :
**FR-A- 2 234 600**
**GB-A- 968 465**
**US-A- 4 573 346**
**Patent Abstracts of Japan, volume 6, No. 116 (P-125)(994), 29 June 1982 & JP-A-5744852**

(73) Titulaire : **TOTAL COMPAGNIE FRANCAISE DES PETROLES Société Anonyme**
**5, rue Michel Ange**
**F-75781 Paris Cédex 16 (FR)**

(72) Inventeur : **CANDAU, Sauveur**
**3, rue de Lens**
**F-67000 Strasbourg (FR)**
Inventeur : **LEMARECHAL, Pierre**
**27, moyenne Corniche**
**F-67210 Obernai (FR)**
Inventeur : **THIRIET, Yves**
**10, rue de la Trinité**
**F-67000 Strasbourg (FR)**
Inventeur : **SCHRANZ, Claude**
**16, rue des Marnes**
**F-924120 Ville-d'Avray (FR)**
Inventeur : **PESNEAU, Bernard**
**39, chemin des Touranies**
**F-91530 Le Val-S.-Germain (FR)**

(74) Mandataire : **Jolly, Jean-Pierre et al**
**Cabinet Jolly, 54, rue de Clichy**
**F-75009 Paris (FR)**

## Description

La présente invention concerne un procédé et un dispositif de mesure de la gélification de produits pétroliers paraffiniques, notamment bruts.

On connaît par le document Patent Abstract of Japan, vol. 6, n° 116 (P - 125) (994), 29 juin 1982, 8 JP - A - 57 44 852, 13 mars 1982, une méthode et un appareil pour la mise en évidence de l'état solide d'un fluide volontairement congelé, contenu dans une conduite de réacteur nucléaire. Ce procédé n'est pas adapté pour la mesure de la gélification des produits pétroliers.

Il est connu que les pétroles bruts paraffiniques sont susceptibles de former des gels à des températures voisines ou inférieures à 40°C. Ce phénomène de gélification, lorsqu'il se produit en cours de production ou de transport, peut conduire à des problèmes de redémarrage des installations. Il est donc d'une importance cruciale de disposer d'une méthode qui permette de déterminer quels sont les bruts susceptibles de gélifier dans un domaine de température donné, et le cas échéant, de déterminer les quantités minimales requises d'agent inhibiteur de gélification ou de produit dopant pour empêcher la formation de gel.

On sait actuellement mesurer le point d'écoulement des bruts (voir la norme ASTM D-97 ou AFNOR T60-103), la méthode consistant à refroidir dans des conditions déterminées du brut dans une éprouvette spéciale munie d'un thermomètre et à examiner tous les trois degrés que la surface du liquide n'est pas figée. La température à laquelle la surface du brut se fige est le point d'écoulement.

On sait également mesurer par analyse calorique différentielle la température de cristallisation commençante du brut, cette dernière correspondant en effet à un phénomène exothermique décelable.

On sait encore mesurer le comportement rhéologique des bruts, les propriétés d'écoulement de ces derniers quittant le comportement newtonien en dessous d'une certaine température lors de l'apparition de cristaux de paraffines en leur sein.

Cependant, aucune de ces données, à savoir : la mesure du point d'écoulement, la mesure de la température de cristallisation commençante et la température de changement du comportement rhéologique ne permettent de savoir assurément que des difficultés d'exploitation ne se produiront pas en cas de refroidissement du brut en dessous de cette température.

La demande vise à répondre à ce besoin d'une connaissance plus sûre relativement à la gélification des bruts paraffiniques en exploitation et propose un procédé de mesure de la gélification des pétroles bruts paraffiniques essentiellement caractérisé en ce que, en vue de déterminer si un brut est sur le point de former un gel susceptible de gêner son exploitation à une température donnée, il consiste à :

- mesurer en fonction de la température et sur une épaisseur donnée la vitesse de propagation et l'amplitude d'une onde ultrasonore dans le brut analysé, et
- à déterminer ensuite la température de transition $T_t$ à laquelle on observe un changement brutal dans la variation en fonction de la température de l'inverse de la vitesse de propagation ;
  à déterminer la valeur du rapport des pentes p de l'inverse de la vitesse de propagation obtenues entre les parties linéaires de la variation thermique de cette dernière, autour dudit point de rupture correspondant à $T_t$, respectivement au-dessous et au-dessus de la température $T_t$ déterminée,
- à déterminer l'écart $\Delta A$ de l'amplitude du signal ultrasonore entre ladite température $T_t$ et une température de référence inférieure choisie, égale par exemple à $T_t$-5°C,
  une rupture de pente significative (p>1) à la dite température $T_t$ et une valeur importante de l'écart A ( A>0,3 dB/cm à $T_t$-5°C) signifiant que la gélification du brut est très probable aux températures inférieures à $T_t$, mais qu'au contraire elle est très peu probable pour une faible variation de l'un de ces paramètres.

La Demanderesse a constaté que la connaissance de p et $\Delta A$ caractérisent l'état physique des produits bruts paraffiniques. Un état de gélification d'un brut implique pour la température inférieure à $T_t$ définie préalablement, le changement de ladite pente thermique ainsi qu'une atténuation importante de l'onde transmise. Inversement, pour les bruts ne présentant pas de risques de gélification, l'un au moins de ces paramètres reste sensiblement inchangé au passage de la température $T_t$.

L'invention concerne également un dispositif de mesure de la vitesse de propagation et de l'amplitude de l'onde ultrasonore. Tout dispositif connu peut servir dans le cadre de l'invention . La gamme de fréquences pratiques utilisées est celle comprise entre 300 kHz et 10 MHz. On peut utiliser par exemple un dispositif qui consiste à mesurer le temps de parcours ou "temps de vol" et l'amplitude d'un signal ultrasonore se propageant à la fréquence de 600 kHz environ, à travers une épaisseur de brut de l'ordre de 1 cm.

Des exemples de réalisation de l'invention sont à présent décrits en référence aux dessins annexés sur lesquels :

- La figure 1 est une vue schématique en coupe transversale d'une cellule de mesure du dispositif de l'invention ;
- La figure 2 représente le schéma-blocs du circuit électronique de ce dispositif ;

- La figure 3 est une vue d'un graphique représentant en fonction de la température, les variations du temps de propagation ou "temps de vol" (homogène à l'inverse de la vitesse de propagation) et de l'amplitude en dB de l'onde ultrasonore dans le brut, et

- La figure 4 représente graphiquement en valeurs réduites $T-T_t$ et $\tau-_T\tau$(où $\tau_t$ est la valeur du temps de vol à $T_t$) les variations du temps de vol de l'onde ultrasonore en fonction de la température pour divers bruts paraffiniques.

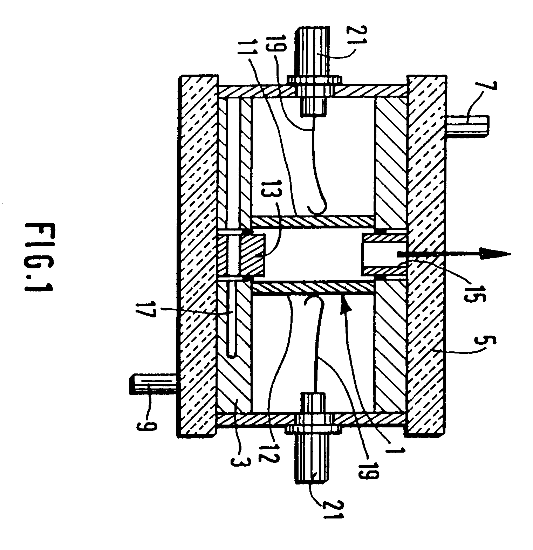

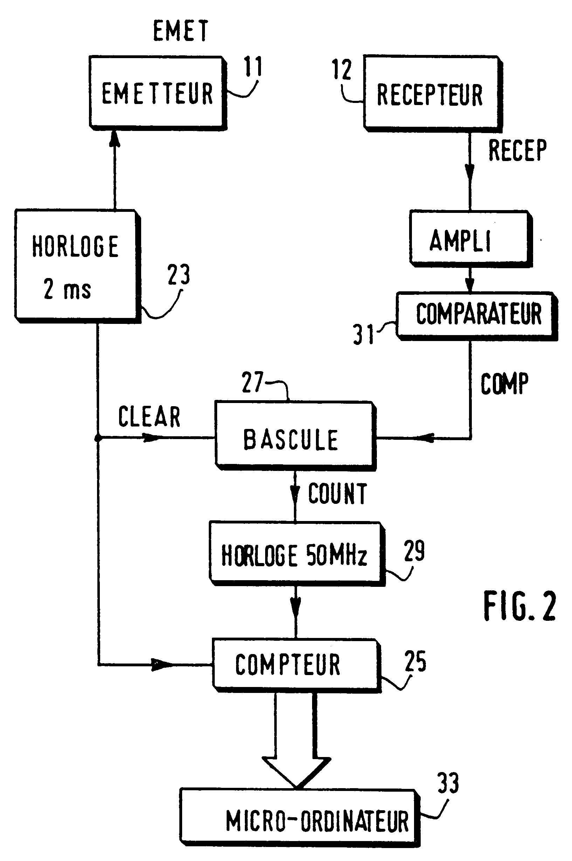

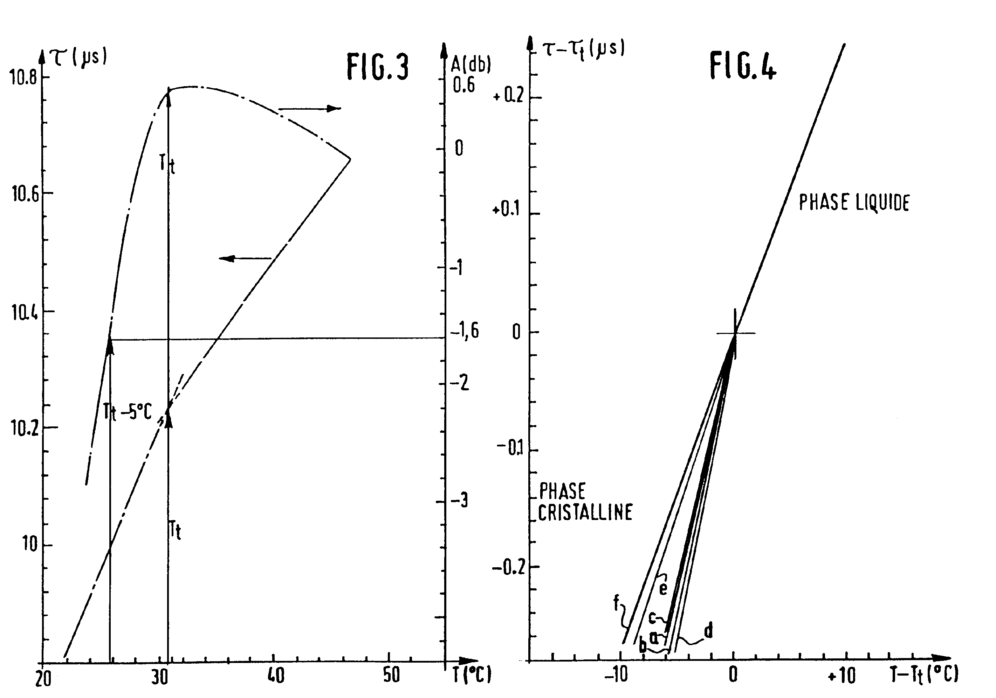

Avec référence à la figure 1, l'appareil de mesure selon l'invention est essentiellement constitué d'une chambre interne centrale 1 recevant le produit brut paraffinique à analyser, cette chambre étant solidaire d'une jaquette métallique thermostatée 3, recouverte d'une paroi 5 isolante thermiquement. La jaquette 3 est maintenue thermostatée par un circuit d'eau entrant par un orifice d'entrée 7 et sortant par un orifice de sortie 9. La chambre interne 1 est cylindrique, son volume interne étant délimité latéralement par deux disques 11 et 12 en céramique piézo-électrique constituant des éléments transducteurs, respectivement émetteur et récepteur ultrasonores de l'appareil, et par une pièce annulaire 13 solidaire de la jaquette 3. Dans une forme de réalisation particulière, la distance entre les disques 11 et 12 est de 14mm et le diamètre interne de la chambre 1 est de 26mm. Le volume de la chambre est par conséquent d'environ 7,5cm$^3$. La pièce annulaire comporte à son niveau supérieur un orifice radial de remplissage 15 d'un volume d'environ 0,5cm$^3$. Le volume total de produit contenu dans la chambre est donc de 8cm$^3$, le trop-plein de l'orifice compensant le retrait du liquide contenu lors du refroidissement. Une sonde thermique 17 est insérée dans l'épaisseur de la pièce annulaire 13 au niveau inférieur. Elle permet de relever la température du pétrole brut à analyser. La paroi isolante externe 5 confère à l'ensemble une stabilité thermique de quelques centièmes de degré. Les éléments transducteurs 11 et 12 sont reliés au circuit electronique de mesure par des fils à ressorts 19 en contact avec les disques piézo-électriques et par l'intermédiaire des fiches raccords 21.

On a représenté à la figure 2 le schéma du circuit électronique permettant la mesure du temps de propagation ou temps de vol de l'onde ultrasonore dans le liquide à analyser contenu dans la chambre interne de l'appareil. Le principe du traitement de signal opéré par ce circuit est le suivant.

Une horloge basse fréquence 23 déclenche toutes les 2 ms le tir d'une impulsion ultrasonore transmise par le disque transducteur émetteur 11 ; simultanément un signal CLEAR en sortie d'horloge, de durée $\tau_o$, est appliqué à l'entrée de remise à zéro d'un compteur binaire 25. Ce signal inhibe le fonctionnement du compteur à une valeur d'origine. Le signal CLEAR a également pour effet d'armer une bascule 27 qui engendre un signal de validation COUNT, autorisant l'activation d'impulsions d'une horloge haute fréquence 29 (50MHz) servant à incrémenter le compteur 25. Ce dernier ne commence à compter qu'à la fin du signal CLEAR et donc après $\tau_o$.

Pendant ce temps, l'onde ultrasonore se propage dans la cellule de mesure puis atteint le transducteur récepteur 12 qui fournit un signal électrique RECEP, reflétant l'énergie ultrasonore reçue après traversée du brut ; le signal RECEP, convenablement amplifié, provoque le basculement d'un comparateur 31 qui désarme la bascule COUNT 27; à ce moment le compteur 25 s'arrête.

La durée du signal COUNT est égale au temps de vol de l'onde ultrasonore dans le brut. L'indication numérique du compteur correspond à la valeur $\tau-\tau_o$, $\tau_o$ ne servant que comme valeur d'origine permettant de cadrer les valeurs de mesure dans l'échelle de mesure permise .

Les sorties numériques du compteur indiquent donc un nombre égal à la différence entre les durées des signaux COUNT et CLEAR, multipliée par la fréquence de l'horloge : ce nombre est transmis à une unité de calcul 33 établissant le calcul d'une valeur moyenne.

Les temps de vol de l'onde ultrasonore mesurés sont de l'ordre de 10 $\mu$s (14mm à 1400m/s environ). Les variations dans la cellule de la vitesse sur le domaine de températures en fonction du brut sont de 10 à 20% et se traduisent par des variations du temps de vol de 1 ou 2 $\mu$s, soit 50 à 100 coups d'horloge. Il suffira de régler la durée $\tau_o$ du signal CLEAR en fonction de cette variation pour cadrer correctement la lecture dans l'échelle de mesure du compteur.

La résolution en temps permise par l'horloge est de 20ns; les diverses instabilités de l'électronique introduisent un bruit de quelques ns. Pour fournir une précision satisfaisante, l'unité de calcul effectue la moyenne de 1000 saisies, diminuant ainsi les perturbations d'un facteur 30 environ ; la précision obtenue est de l'ordre de 1 ns soit 1/10.000 du temps de vol.

En ce qui concerne l'atténuation ultrasonore de l'onde de propagation dans le brut, elle est mesurée à partir de la variation de l'amplitude du signal reçu, une fois prises en compte les variations thermiques du couplage des disques transducteurs avec le milieu étudié.

On a représenté graphiquement à la figure 3, les variations du temps de vol et de l'atténuation ultrasonore d'un brut typique, le brut n° 2. Le brut comporte une température de transition en dessous de laquelle il est susceptible de gélifier et que l'on observe au point de rupture de la courbe de temps de vol à une valeur proche de 30°C. De part et d'autre de ce point, les variations du temps de vol sont linéaires, le segment correspondant

à $T<T_t$ ayant une pente plus importante que celui correspondant à $T>T_t$. Le rapport p précité des pentes pour ces segments est donc nettement supérieur à 1.

En ce qui concerne l'atténuation ultrasonore, correspondant aux variations de l'amplitude du signal dont l'échelle en dB est représentée à droite, on observe, en dessous d'une température voisine de $T_t$ une diminution rapide de l'amplitude, due à une augmentation des pertes et correspondant à la gélification du brut. On voit donc bien que les paramètres cités p et A sont en corrélation avec l'état physique du brut.

On a représenté à la figure 4, les variations du temps de vol (inversement proportionnel à la vitesse de propagation) en fonction de la température des bruts paraffiniques n°1:a, n°2:b, n°3:c, n°4:d, n°5:e et n°6:f. On constate que les variations du temps de vol en fonction de la température de tous ces bruts sont linéaires pour $T<T_t$ avec des pentes différentes selon les bruts. Elles sont également linéaires pour $T>T_t$ (phase liquide) mais les pentes sont alors toutes identiques.

Les bruts n°1, n°2, n°3 et n°4 sont caractérisés par une rupture de pente à une température caractéristique $T_t$, et par une variation à nouveau linéaire mais de pente p plus forte pour $T<T_t$ (phase cristalline).

Pour le brut n°5, la rupture de pente n'est que peu prononcée ($p \simeq 1$), tandis que pour celui de n°6 elle n'est pas discernable. Ces deux derniers bruts ne présentent pas dans les faits de risques d'obstruction des conduites en exploitation. Ceci montre bien que p est l'un des paramètres caractéristiques de la formation de gel et que s'il est sensiblement égal à 1, on a très peu de chances d'obtenir un gel.

On a récapitulé dans le tableau I ci-dessous les valeurs de p et la différence $\Delta$ A respectivement entre $T_t$ et $T_t$-5°C pour les bruts cités.

## TABLEAU I

| Brut | $T_t$ (°C) | p | $\Delta$ A (db) |
|---|---|---|---|
| Brut n°1 | 37,5 | 1,6 | 0,5 |
| Brut n°2 | 31 | 1,8 | 2,1 |
| Brut n°3 | 36 | 1,5 | 1,0 |
| Brut n°4 | 36 | 1,9 | 2,3 |
| Brut n°5 | 22 | 1,1 | 0 |
| Brut n°6 | -- | 1,0 | 0 |
| n°2+200 ppM | 31 | 1,4 | 0 |
| n°2+400 ppM | 31 | 1,4 | 0 |

Dans le cas des bruts n°5 et n°6, la différence $\Delta$ A reste proche de zéro, ce qui confirme bien l'indication obtenue par p.

Dans le tableau II ci-dessous, on a résumé les résultats d'essais pour les différents bruts étudiés ainsi que pour des bruts dopés, et en particulier deux échantillons de brut n°2 dopés avec respectivement 200 et 400 ppM d'une produit dépresseur de point de gélification.

TABLEAU II

| Bruts | Observations | Résultat du test |
|---|---|---|
| Brut n°6<br>Brut n°5 | Pas de transition<br>faible variation de A<br><br>$p \simeq 1$   $A \simeq 0$ | Pas de gélifi-<br>cation possible<br>dans le domaine<br>de température<br>étudié |
| Brut n°1<br>Brut n°2<br>Brut n°4<br>Brut n°3 | Transition marquée<br>forte variation de A<br><br>$p > 1$<br>$\Delta A$ important pour $T < T_t$ | Gélification<br>possible<br>pour $T < T_t$ |
| Brut n°2<br>+ dopant | Transition nette<br>faible variation de A<br><br>$p > 1$   $A \simeq 0$ | Pas de gélifi-<br>cation possible<br>dans le domaine<br>de température<br>étudié |

La présence du dopant est caractérisée par l'absence d'atténuation ($\Delta A \simeq 0$) de l'onde ultrasonore de propagation.

Par conséquent, la méthode vaut également pour les bruts dopés dont on veut prévenir la gélification en ajustant les quantités de produit dopant jusqu'à obtenir une valeur $\Delta A$ sensiblement nulle.

La méthode pourrait s'appliquer à d'autres matériaux que les bruts paraffiniques et par exemple aux coupes pétrolières paraffiniques, et de façon générale aux produits d'origine pétrolière tels que les gaz naturels à condensat (hydrates de gaz naturels dont on veut prévenir le dépôt au sein des canalisations en particulier par ajout d'inhibiteurs spécifiques), certains produits inhérents aux hydrocarbures, asphalthènes par exemple dont on veut prévenir la précipitation en leur sein, paraffines, cires etc. En outre, dans l'exemple décrit on a procédé sans condition de pression particulière, l'examen se faisant à la pression atmosphérique mais il entre également dans le cadre du principe de l'invention de pouvoir faire varier ce paramètre notamment pour l'étude des préventions des dépôts précités, la cellule de mesure étant mise en pression par des moyens classiques appropriés.


**Revendications**

1. Procédé de mesure de la gélification des pétroles bruts paraffiniques, caractérisé en ce que, en vue de déterminer si un pétrole brut est sur le point de former un gel susceptible de gener son exploitation à une température donnée, il consiste à :
   - mesurer en fonction de la température et sur une épaisseur donnée la vitesse de propagation et l'amplitude d'une onde ultrasonore dans le pétrole brut analysé,
   - à déterminer ensuite la température de transition $T_t$ à laquelle on observe un changement brutal dans la variation en fonction de la température de l'inverse de la vitesse de propagation ;
   - à déterminer la valeur du rapport des pentes p de l'inverse de la vitesse de propagation obtenues entre les parties linéaires de la variation thermique de cette dernière, autour dudit point de rupture correspondant à $T_t$, respectivement au-dessous et au-dessus de la température $T_t$ déterminée,
   - à déterminer l'écart $\Delta A$ de l'amplitude du signal ultrasonore entre la température $T_t$ et une température de référence inférieure choisie, une rupture de pente significative ($p > 1$) à la dite température $T_t$ et une valeur significative de l'écart $\Delta A$, supérieure à une limite établie à $T_t$, signifiant que la gélification du pétrole brut est très probable aux températures inférieures à $T_t$, mais qu'au contraire elle est très peu probable pour une faible variation de l'un de ces paramètres.

2.  Procédé de mesure de la gélification selon la revendication 1, caractérisé en ce que ladite fréquence de l'onde ultrasonore propagée dans le pétrole brut à analyser est choisie dans une plage comprise entre 300kHz et 10 MHz.

3.  Procédé de mesure de la gélification selon l'une des revendications précédentes, caractérisé en ce que la fréquence de l'onde ultrasonore est de 600kHz et la distance de propagation de l'onde dans le pétrole brut à analyser est de 1cm.

4.  Dispositif pour la mise en oeuvre du procédé défini selon l'une des revendications précédentes, caractérisé en ce qu'il comprend une chambre interne (1) recevant le pétrole brut à analyser, solidaire d'une jaquette métallique thermostatée (3), elle même recouverte d'une paroi isolante thermiquement (5), la propagation ultrasonore étant effectuée et mesurée entre deux disques transducteurs ultrasonores (11, 12) respectivement émetteur et récepteur, disposés en vis à vis et constituant les parois latérales de ladite chambre (1), et un circuit électronique classique de traitement du signal ultrasonore relié auxdits disques transducteurs permettant de :
    -   mesurer en fonction de la température et sur une épaisseur donnée la vitesse de propagation et l'amplitude d'une onde ultrasonore dans le pétrole brut analysé,
    -   à déterminer ensuite la température de transition $T_t$ à laquelle on observe un changement brutal dans la variation en fonction de la température de l'inverse de la vitesse de propagation ;
    -   à déterminer la valeur du rapport des pentes p de l'inverse de la vitesse de propagation obtenues entre les parties linéaires de la variation thermique de cette dernière, autour dudit point de rupture correspondant à $T_t$, respectivement au-dessous et au-dessus de la température $T_t$ déterminée,
    -   à déterminer l'écart $\Delta A$ de l'amplitude du signal ultrasonore entre la température $T_t$ et une température de référence inférieure choisie,

5.  Dispositif pour la mise en oeuvre du procédé selon la revendication 4, caractérisé en ce que les disques transducteurs (11) sont en céramique piézo-électrique.

6.  Dispositif pour la mise en oeuvre du procédé selon l'une des revendications 4 et 5, caractérisé en ce que la distance entre lesdits disques transducteurs (11, 12) est égale à 14mm.

7.  Dispositif selon l'une des revendications 4-6, caractérisé en ce que les disques transducteurs (11,12) sont reliés extérieurement à des fils en forme de ressorts externes (19) convenablement raccordés à un circuit électronique de mesure du temps et de l'amplitude de propagation de l'onde ultrasonore propagée au sein de la chambre interne.

8.  Dispositif selon l'une des revendications 4 à 7, caractérisé en ce qu'il comporte une unité de calcul (33), par exemple microprocesseur, effectuant le calcul d'une valeur moyenne desdits paramètres p et A, sur un nombre de saisies suffisamment grand pour obtenir une précision de l'ordre de 1/10.000 du temps de vol, l'atténuation ultrasonore étant en outre déterminée en tenant compte des variations thermiques du couplage des transducteurs (11) avec le milieu étudié.

9.  Dispositif selon l'une des revendications 4 à 7, caractérisé en ce que la cellule de mesure ou chambre interne (1) est mise en pression.

10.  Application du procédé selon l'une des revendications 1 à 3 au dopage des pétroles bruts paraffiniques en vue de prévenir leur gélification, la quantité de produit dopant étant ajustée jusqu'à obtenir une valeur A de l'atténuation sensiblement nulle.

11.  Application du procédé selon l'une des revendications 1 à 3 au suivi de la cristallisation de coupes pétrolières, et de façon générale aux produits d'origine pétrolière, tels que les gaz naturels à condensat, certains produits inhérents aux hydrocarbures, asphaltènes par exemple dont on veut prévenir la précipitation en leur sein, paraffines et cires.

**Patentansprüche**

1.  Verfahren zur Messung der Gelbildung von paraffinischen Rohölen, dadurch gekennzeichnet, daß es zur Bestimmung, ob ein Rohöl sich an einem Punkt der Gelbildung befindet, der bei einer gegebenen Temperatur Förderung oder Transport des Rohöls zu beeinträchtigen geeignet ist, aus folgenden Schritten besteht:

- Messen der Ausbreitungsgeschwindigkeit und Amplitude einer Ultraschallwelle im analysierten Rohöl in Abhängigkeit von der Temperatur und über eine gegebene Breite (Dicke);
- nachfolgendes Ermitteln der Übergangstemperatur $T_t$, bei der im temperaturabhängigen Änderungsverlauf des Kehrwertes der Ausbreitungsgeschwindigkeit eine abrupte Richtungsänderung auftritt;
- Ermitteln des Verhältniswertes der Steigungen p des Kehrwertes der Ausbreitungsgeschwindigkeit, die zwischen den linearen Bereichen der thermischen Änderung der letzteren um den $T_t$ entsprechenden Änderungspunkt bzw. unterhalb bzw. oberhalb der ermittelten Temperatur $T_t$ auftreten;
- Ermitteln der Abweichung $\Delta A$ zwischen der bei der Temperatur $T_t$ und der bei einer gewählten niedrigeren Bezugstemperatur auftretenden Amplitude des Ultraschallsignals, wobei ein Abbruch der bei der genannten Temperatur $T_t$ signifikanten Steigung (p > 1) und ein für die Abweichung $\Delta A$ signifikanter Wert oberhalb einer bei $T_t$ festgelegten Grenze darauf hinweisen, daß die Gelbildung des Rohöls bei Temperaturen unterhalb von $T_t$ sehr wahrscheinlich, dagegen durch geringfügige Veränderung eines der genannten Parameter sehr unwahrscheinlich ist.

2. Verfahren nach Anspruch 1, dadurch gekennzeichnet, daß die Frequenz der sich in dem zu analysierenden Rohöl ausbreitenden Ultraschallwelle aus einem Bereich zwischen 300 KHz und 10 MHz gewählt wird.

3. Verfahren nach Anspruch 1 oder 2, dadurch gekennzeichnet, daß die Frequenz der Ultraschallwelle 600 KHz und die Ausbreitungsstrecke der Welle in dem zu analysierenden Rohöl 1 cm beträgt.

4. Vorrichtung zur Durchführung des Verfahrens nach einem der vorhergehenden Ansprüche, dadurch gekennzeichnet, daß eine das zu analysierende Rohöl aufnehmende Innenkammer (1) in einem auf konstanter Temperatur gehaltenen metallischen Schutzbehälter (3) befestigt ist, der äußere Wärmeisolierwandungen (5) aufweist, wobei die Ultraschallausbreitung zwischen zwei als Sender und Empfänger einander gegenüberliegenden und die Seitenwände der Kammer (1) bildenden Ultraschall-Übertragerscheiben (11, 12) durchgeführt und gemessen wird, und wobei eine an die Übertragerscheiben angeschlossene herkömmliche Elektronikschaltung zur Verarbeitung des Ultraschallsignals folgende Schritte ermöglicht:

- Messen der Ausbreitungsgeschwindigkeit und Amplitude einer Ultraschallwelle im analysierten Rohöl in Abhängigkeit von der Temperatur und über eine gegebene Breite (Dicke);
- nachfolgendes Ermitteln der Übergangstemperatur $T_t$, bei der im temperaturabhängigen Änderungsverlauf des Kehrwertes der Ausbreitungsgeschwindigkeit eine abrupte Richtungsänderung auftritt;
- Ermitteln des Verhältniswertes der Steigungen p des Kehrwertes der Ausbreitungsgeschwindigkeit, die zwischen den linearen Bereichen der thermischen Änderung der letzteren um den $T_t$ entsprechenden Änderungspunkt bzw. unterhalb bzw. oberhalb der ermittelten Temperatur $T_t$ auftreten;
- Ermitteln der Abweichung $\Delta A$ zwischen der bei der Temperatur $T_t$ und der bei einer gewählten niedrigeren Bezugstemperatur auftretenden Amplitude des Ultraschallsignals.

5. Vorrichtung nach Anspruch 4, dadurch gekennzeichnet, daß die Übertragerscheiben (11) aus piezoelektrischem Keramikmaterial bestehen.

6. Vorrichtung nach Anspruch 4 oder 5, dadurch gekennzeichnet, daß der Abstand zwischen den Übertragerscheiben (11, 12) 14 mm beträgt.

7. Vorrichtung nach einem der Ansprüche 4 bis 6, dadurch gekennzeichnet, daß die Übertragerscheiben (11, 12) an ihren Außenseiten mit Drähten in Form äußerer Federn (19) in Verbindung stehen, die auf geeignete Weise an eine Elektronikschaltung zur Messung der Ausbreitungszeit und -amplitude der sich innerhalb der Innenkammer fortpflanzenden Ultraschallwelle angeschlossen sind.

8. Vorrichtung nach einem der Ansprüche 4 bis 7, dadurch gekennzeichnet, daß sie eine Recheneinheit (33), z.B. Mikroprozessor aufweist, die einen Mittelwert der Parameter p und $\Delta A$ aus einer ausreichend großen Anzahl von Meßdaten mit einer Genauigkeit in der Größenordnung von 1/10.000 der Wellenlaufzeit berechnet, wobei außerdem die Ultraschalldämpfung unter Berücksichtigung der thermischen Änderungen zwischen der Ankopplung der Übertrager (11) an das zu untersuchende Medium ermittelt wird.

9. Vorrichtung nach einem der Ansprüche 4 bis 7, dadurch gekennzeichnet, daß die Meßzelle oder Innenkammer (1) unter Druck gesetzt ist.

10. Anwendung des Verfahrens nach einem der Ansprüche 1 bis 3 zum Dopen paraffinischer Rohöle zwecks Verhinderung ihrer Gelbildung, wobei die Menge des Dopingmittels dahingehend eingestellt wird, bis ein im wesentlichen bei Null liegender Dämpfungswert A erreicht ist.

11. Anwendung des Verfahrens nach einem der Ansprüche 1 bis 3 bei der Verfolgung der Kristallisation von Erdölmengen oder -chargen und im allgemeinen bei aus Erdöl entstehenden Produkten, wie natürlichen Kondensatgasen, bestimmten Kohlenwasserstoffen immanenten Stoffen, Asphaltenen beispielsweise, in denen eine Ausscheidung oder ein Niederschlag vermieden werden soll, Paraffinen und Wachsen.

## Claims

1. Process for measuring the gelling of paraffinic crude oils, characterised in that, with a view to determining whether a crude oil is on the point of forming a gel liable to interfere with its use at a given temperature, it consists in:
   - measuring, as a function of temperature and over a given thickness, the speed of propagation and the amplitude of an ultrasonic wave in the crude oil analysed,
   - in next determining the transition temperature $T_t$ at which an abrupt change is observed in the variation as a function of temperature of the reciprocal of the speed of propagation;
   - in determining the value of the ratio of the slopes p of the reciprocal of the speed of propagation which are obtained between the linear parts of the thermal variation of the said speed, about the said break point corresponding to $T_t$, below and above the determined temperature $T_t$ respectively,
   - in determining the difference $\Delta A$ in the amplitude of the ultrasonic signal between the temperature $T_t$ and a chosen lower reference temperature, a significant break in slope (p>1) at the said temperature $T_t$ and a significant value of the difference $\Delta A$, higher than a limit established at $T_t$, meaning that gelling of the crude oil is highly probable at temperatures lower than $T_t$, but that, on the contrary, it is very improbable in the case of a very small variation in one of these parameters.

2. Process for measuring gelling according to Claim 1, characterised in that the said frequency of the ultrasonic wave propagated in the crude oil to be analysed is chosen in a range of between 300 kHz and 10 MHz.

3. Process for measuring gelling according to either of the preceding claims, characterised in that the frequency of the ultrasonic wave is 600 kHz and the distance of propagation of the wave in the crude oil to be analysed is 1 cm.

4. Device for making use of the process defined according to one of the preceding claims, characterised in that it comprises an internal chamber (1) receiving the crude oil to be analysed, integrally attached to a thermostatted metal jacket (3) itself covered with a thermally insulating wall (5), the ultrasonic propagation being performed and measured between two ultrasonic transducer discs (11, 12), transmitter and receiver respectively, which are arranged facing each other and forming the side walls of the said chamber (1), and a conventional electronic circuit for processing the ultrasonic signal linked to the said transducer discs making it possible:
   - to measure, as a function of temperature and over a given thickness, the speed of propagation and the amplitude of an ultrasonic wave in the crude oil analysed,
   - to next determine the transition temperature $T_t$ at which an abrupt change is observed in the variation as a function of the temperature of the reciprocal of the speed of propagation;
   - to determine the value of the ratio of the slopes p of the reciprocal of the speed of propagation which are obtained between the linear parts of the thermal variation of the said speed, about the said break point corresponding to $T_t$, below and above the determined temperature $T_t$ respectively,
   - to determine the difference $\Delta A$ in the amplitude of the ultrasonic signal between the temperature $T_t$ and a chosen lower reference temperature.

5. Device for making use of the process according to Claim 4, characterised in that the transducer discs (11) are made of piezoelectric ceramic.

6. Device for making use of the process according to either of Claims 4 and 5, characterised in that the distance between the said transducer discs (11, 12) is equal to 14 mm.

7. Device according to one of Claims 4-6, characterised in that the transducer discs (11, 12) are connected externally to wires in the form of external springs (19) suitably joined to an electronic circuit for measuring the time and the amplitude of propagation of the ultrasonic wave propagated within the internal chamber.

8. Device according to one of Claims 4 to 7, characterised in that it comprises a calculating unit (33), for example a microprocessor, performing the calculation of a mean value of the said parameters p and A over a sufficiently large number of readings to obtain an accuracy of the order of 1/10,000 of the flight time, the ultrasonic attenuation being furthermore determined by taking into account the thermal variations in the coupling of the transducers (11) with the medium being studied.

9. Device according to one of Claims 4 to 7, characterised in that the measurement cell or internal chamber (1) is pressurised.

10. Application of the process according to one of Claims 1 to 3 to the doping of paraffinic crude oils with a view to preventing their gelling, the quantity of doping product being adjusted until a substantially zero attenuation value A is obtained.

11. Application of the process according to one of Claims 1 to 3 to the monitoring of the crystallisation of petroleum cuts and generally to products originating from oil, such as natural gases containing condensate, some products inherent in hydrocarbons, asphaltines, for example, in which it is desired to prevent the precipitation of paraffins and waxes therein.

FIG.1

EMET

EMETTEUR  11

12  RECEPTEUR

RECEP

HORLOGE
2 ms  23

AMPLI

COMPARATEUR
31

27  COMP

CLEAR  BASCULE

COUNT

HORLOGE 50MHz  29

FIG. 2

COMPTEUR  25

MICRO-ORDINATEUR  33

FIG.3

FIG.4

PHASE LIQUIDE

PHASE CRISTALLINE

EP 0 480 980 B1